(19) Europäisches Patentamt
European Patent Office
Office européen des brevets

(11) Veröffentlichungsnummer: **0 064 205**
**B1**

(12) # EUROPÄISCHE PATENTSCHRIFT

(45) Veröffentlichungstag der Patentschrift: **05.12.84**

(51) Int. Cl.³: **C 01 B 33/28**, B 01 J 29/28

(21) Anmeldenummer: **82103256.2**

(22) Anmeldetag: **19.04.82**

(54) **Kristallines Alumosilicat, Verfahren zu dessen Herstellung sowie dessen Verwendung zur katalytischen Umwandlung von Methanol und/oder Dimethylether in Kohlenwasserstoffe.**

(30) Priorität: **30.04.81 DE 3117135**

(43) Veröffentlichungstag der Anmeldung: **10.11.82 Patentblatt 82/45**

(45) Bekanntmachung des Hinweises auf die Patenterteilung: **05.12.84 Patentblatt 84/49**

(84) Benannte Vertragsstaaten: **BE DE FR GB IT NL**

(56) Entgegenhaltungen:
**DE - A - 2 909 927**
**US - A - 3 894 105**
**US - A - 3 894 107**
**US - A - 3 911 041**
**US - A - 4 025 575**
**US - A - 4 104 151**

(73) Patentinhaber: **BAYER AG, Konzernverwaltung RP Patentabteilung, D-5090 Leverkusen 1 Bayerwerk (DE)**

(72) Erfinder: **Puppe, Lothar, Dr., Gisbert-Cremer-Strasse 45a, D-5090 Leverkusen (DE)**
Erfinder: **Weisser, Jürgen, Dr., Südstrasse 15, D-4047 Dormagen 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 064 205 B1

## Beschreibung

Gegenstand der Erfindung ist ein neues zeolithartiges Material, das nachfolgend als PSH-3 bezeichnet wird, sowie ein Verfahren zur Herstellung desselben und dessen Verwendung als Katalysator bei der Herstellung von Kohlenwasserstoffen aus Methanol und/oder Dimethylether.

Unter Zeolithe versteht man wasserhaltige Gerüstsilikate. Sie lassen sich als starres dreidimensionales Gitter von $SiO_4$- und $AlO_4$-Tetraedern beschreiben, die durch gemeinsame Sauerstoffatome verknüpft sind. Die Ladung des $AlO_4$-Tetraeders ist negativ und wird beispielsweise durch ein Alkali- bzw. Erdalkalikation ausgeglichen. Die Hohlräume der kristallinen Aluminosilicate sind mit Wassermolekülen besetzt, die entfernt werden können, ohne dass das Gerüst zusammenbricht.

Es ist bekannt, organische Stickstoffverbindungen für Zeolithsynthesen einzusetzen. Hierbei werden vor allem $SiO_2$-reiche Zeolithtypen erhalten. Am bekanntesten sind in letzter Zeit Zeolithe mit der Bezeichnung ZSM-5 bzw. ZSM-11 geworden, die besondere katalytische Eigenschaften aufweisen. Einen Überblick der Zeolithe, die mit Hilfe organischer Kationen erhalten wurden, findet man in „Zeolite Molekular Sieves" von D.W. Breck (Wiley, „Interscience", 1974), S. 304 bis 312 und 348 bis 378.

Gegenstand der vorliegenden Erfindung ist ein neues Zeolithmaterial, also ein kristallines Aluminosilicat, mit einer Zusammensetzung entsprechend der Formel (in Molverhältnissen der Oxide)

$$M_{2/n}O \cdot Al_2O_3 \cdot (20\text{-}150)\ SiO_2$$

wobei M ein Kation der Wertigkeit n ist und mit einem Pulverröntgendiagramm wie in Tabelle 1 angegeben.

Das erfindungsgemässe kristalline Aluminosilicat kann aus einer Synthesemischung erhalten werden, die eine Kieselsäure, eine Al-Verbindung, Alkali und Hexamethylenimin enthält.

Der Zeolith PSH-3 erweist sich anhand von Röntgenbeugungsdaten und anderen Charakteristiken als unterschiedlich von allen bisher bekannten natürlichen und synthetischen Zeolithen.

Der erfindungsgemässe Zeolith PSH-3 hat folgende oxidische Zusammensetzung

$$M_{2/n}O \cdot Al_2O_3 \cdot (20\text{-}150)\ SiO_2$$

und besitzt ein Pulverröntgendiagramm, wie es im wesentlichen in der Tabelle I aufgeführt wird.

Vorzugsweise beträgt das $SiO_2/Al_2O_3$-Verhältnis 30-100. Der Zeolith PSH-3 hat in der bevorzugt synthetisierten Form folgende Zusammensetzung

$$(0{,}1\text{-}0{,}8)\ Na_2O\ (0{,}2\text{-}0{,}8)\ R_2O{:}Al_2O_3{:}(20\text{-}150)\ SiO_2$$

R ist ein sich vom Hexamethylenimin ableitendes organisches stickstoffhaltiges Kation.

Entsprechend dem Herstellungsverfahren wird ein Gemisch aus einer Mineralsäure, $Na_2O$, $SiO_2$, $Al_2O_3$, Hexamethylenimin und Wasser gebildet, das die Zusammensetzung in Molverhältnissen von Oxiden in den folgenden Bereichen aufweist

$SiO_2/Al_2O_3$ = 10-200
$OR^-/SiO_2$ = 0,05-1,0
$R/Na_2O$ = 0,5-5,0

wobei R = Hexamethylenimin.

Hexamethylenimin ist ein siebengliedriger Heterocyclus mit einem Stickstoffatom.

Vorzugsweise wird die Synthesemischung in folgenden Bereichen gehalten

$SiO_2/Al_2O_3$ = 20-150
$OH^-/SiO_2$ = 0,15-0,5
$R/Na_2O$ = 1,0-3,0

Der kristalline Aluminosilicatzeolith PSH-3 hat folgende charakteristische Linien im Röntgendiagramm, die in der Tabelle I angeführt sind.

Die Werte wurden mit der Kα-Strahlung des Kupfers ermittelt. Die relativen Intensitäten $100\ I/I_o$, wobei $I_o$ die Intensität der stärksten Linie und d der Netzebenenabstand in Å ist, wurden aus dem Schreiberdiagramm ermittelt.

In der Tabelle I werden die relativen Intensitäten wie folgt beschrieben

60-100 sehr stark
40- 60 stark
20- 40 mittel
0- 20 schwach

*Tabelle I*

| d-Wert | Intensität |
|---|---|
| 12,63 | sehr stark |
| 10,92 | mittel |
| 8,84 | sehr stark |
| 6,86 | schwach |
| 6,15 | stark |
| 5,50 | schwach |
| 4,91 | schwach |
| 4,60 | schwach |
| 4,39 | schwach |
| 4,09 | schwach |
| 3,91 | mittel |
| 3,75 | schwach |
| 3,56 | schwach |
| 3,41 | sehr stark |
| 3,30 | schwach |
| 3,19 | schwach |
| 3,11 | schwach |
| 2,836 | schwach |
| 2,694 | schwach |
| 2,592 | schwach |
| 2,392 | schwach |
| 2,206 | schwach |
| 2,122 | schwach |
| 2,036 | schwach |
| 1,973 | schwach |
| 1,873 | schwach |
| 1,855 | schwach |

Die Gleichgewichtsadsorptionen des kristallinen Aluminosilicats PSH-3 für einige Verbindungen sind in der folgenden Tabelle II aufgeführt.

*Tabelle II*

Adsorbat in g/100 g Adsorbens

| | $H_2O$ | n-Hexan | Cyclohexan |
|---|---|---|---|
| PSH-3 | 7,7 | 6,9 | 6,1 |

Die ursprünglichen Kationen des Zeoliths können durch Ionenaustausch mit anderen Kationen nach bereits bekannten Techniken ersetzt werden. Bevorzugt erfolgt der Austausch nach dem Calcinieren des Zeoliths.

Der krist. Zeolith wird durch Kristallisieren eines Reaktionsgemisches einer Zusammensetzung in den folgenden oxidischen Molverhältnissen erhalten

$SiO_2/Al_2O_3 = 10\text{-}200$
$OH^-/SiO_2 = 0,05\text{-}1,0$
$R/Na_2O = 0,5\text{-}5,0$

wobei R = Hexamethylenimin $(CH_2)_6NH$.

Die Reaktionsdauer beträgt bei einer Temperatur von 80 bis 180° C ca. 12 bis 144 h, bevorzugt 110 bis 150° C 24 bis 96 h.

Das erhaltene kristalline Produkt wird von der Mutterlauge abgetrennt, mit Wasser auf einen pH-Wert im ablaufenden Wasser von 8-10 gewaschen und bei 120° C getrocknet.

Wird der Zeolith PSH-3 als Adsorbens oder bevorzugt als Katalysator eingesetzt, so erfolgt unter Anwendung üblicher Techniken eine Entfernung der org. Verbindungen und des Wassers aus den Hohlräumen. Dies geschieht durch Erhitzen auf 300 bis 600° C in einer Atmosphäre wie Luft, Stickstoff, Ammoniak usw. innerhalb von 1 bis 24 h.

Die Ausgangsstoffe für die Herstellung des erfindungsgemässen Zeoliths sind Materialien, die die geeigneten oxidischen Verbindungen liefern. Hierfür kommen beispielsweise in Frage Na-Aluminat, Al-Sulfat, $Al(OH)_3$, $Al_2O_3$, Kieselsol, $SiO_2$-Füllstoffe, Na-Silikate, NaOH sowie Mineralsäuren wie z.B. $H_2SO_4$ und als org. Verbindung Hexamethylenimin.

Für eine weitere Verwendung der krist. Zeolithe als Katalysator ist im allgemeinen eine Modifizierung notwendig, wie sie durch bereits bekannte Techniken des Ionenaustausches erfolgen kann. So erhält man die H-Form des Zeoliths entweder durch Einwirken von verdünnten Säuren bzw. durch Austausch mit Ammoniumsalzen und anschliessender Calcinierung.

Weiterhin sind Austausche mit Übergangsmetallionen, z.B. der Gruppe VIIa, VIIIa, Ib und IIb möglich. Zusätzliche Austausche bzw. Modifikationen mit Hauptgruppenelementen sind durchführbar.

Für die erfindungsgemässe Anwendung wird der Zeolith PSH-3 vorzugsweise in der dehydratisierten H-Form eingesetzt. Ebenso ist es vorteilhaft, wenn ein Gemisch der H-Form mit einer metallausgetauschten Form, beispielsweise der Mg-, Mn-, Ba- und/oder Seltenerdform zu einem Katalysator verarbeitet wird.

Der Zeolith PSH-3 kann in einer Vielzahl von Teilchengrössen zu Katalysatoren verarbeitet werden.

Je nach verfahrenstechnischer Durchführung (Flugstaubverfahren, Wirbelschichtverfahren, Wanderbettverfahren, Festbettverfahren) können die Teilchen in Form eines Pulvers, eines Granulats oder als Formkörper eingesetzt werden.

Zur Herstellung der verschiedenen Anwendungsformen, die nach bekannten Techniken geschehen kann, ist es u. U. erforderlich, dass man den vorstehend beschriebenen Zeolith PSH-3 mit Inertmaterialien und/oder Bindemitteln zu technisch brauchbaren Katalysatoren verarbeitet.

Beispielsweise sind für die Herstellung technischer Katalysatoren auf Basis Zeolith PSH-3 die Inertmaterialien bzw. Bindemittel, Tone, Tonerden, Bentonite, Smectite, natürlich vorkommende oder synthetisch hergestellte Metalloxide, beispielsweise Aluminiumoxid, Siliciumdioxid, Titandioxid, Zirkoniumdioxid, Thoriumoxid, Zinnoxid, Zinkoxid, Magnesiumoxid und/oder Berylliumoxid geeignet.

Die relativen Anteile der Zeolithkomponente können im Hinblick auf das verwendete Trägermaterial in weiten Bereichen variiert werden; nämlich im Bereich von 1 bis 99% und bevorzugt 10 bis 80 Gew.-%, bezogen auf die trockene Katalysatormasse.

Der vorstehend beschriebene Zeolith PSH-3 eignet sich als Katalysator für die Umwandlung von niederen aliphatischen Alkoholen und/oder Ethern, vorzugsweise Methanol und/oder Dimethylether, in überwiegend aliphatisch ungesättigte Kohlenwasserstoffe mit niedrigem Aromatengehalt.

Beispielsweise ist bekannt, dass sich Methanol und/oder Dimethylether an Zeolithen vom Typ ZSM-5 oder ZSM-11 in Wasser und eine aromatenreiche Kohlenwasserstoff-Fraktion umwandeln lässt.

Beispielsweise ist in der US-Patentschrift Nr. 3894107 beschrieben, dass das durch Zeolithkatalyse aus Methanol erhaltene Kohlenwasserstoffgemisch zu 40% und mehr aus Aromaten bestehen kann. Weiterhin lehrt die US-Patentschrift Nr. 3894105, dass die aus Methanol erhaltenen Aromaten einen beträchtlichen Anteil an 1,2,4,5-Tetramethylbenzol (Durol) aufweisen. Bei Verwendung des aus Methanol erhaltenen Kohlenwasserstoffgemisches als Vergaserkraftstoff ist der Durolanteil in der Aromatenfraktion nachteilig.

Durol ist in Kohlenwasserstoffen bei Raumtemperatur schwerlöslich, und es neigt aufgrund seines hohen Schmelzpunktes (79,2° C) zum Auskristallisieren. Es ist offensichtlich, dass dieses Auskristallisieren Störungen beim Betrieb von Verbrennungsmotoren verursachen kann, die mit aus Methanol hergestelltem Kraftstoff betrieben werden. Es hat zahlreiche Versuche gegeben, die Umwandlung von Methanol an Zeolithen so zu steuern, dass die flüssigen Reaktionsprodukte nur wenig Durol bzw. allgemein wenig Aromaten enthalten.

Beispielsweise lässt sich durch Modifizieren eines ZSM-5 Zeolithkatalysators mit organischen Verbindungen des Phosphors die Katalysatoraktivität derartig herabsetzen, dass die Reaktionsprodukte vorwiegend aliphatischer Natur sind. Allerdings ist die Herstellung der Katalysatoren aufwendig und in technischer Hinsicht aufgrund der Toxizität von organischen Phosphinen, Phosphinoxiden, Phosphorsäurederivaten etc. nicht unbedenklich (US-Patentschrift Nr. 3911041).

Weiterhin wurde versucht, die Bildung alkylierter Aromaten durch Einstellung bestimmter verfahrenstechnischer Parameter, z.B. Herabsetzung des Partialdruckes des Einsatzmethanols, zu verhindern. Wie beispielsweise aus der US-Patentschrift Nr. 4025575 hervorgeht, lässt sich durch Verdünnung des als Einsatz dienenden Methanols mit inerten Gasen die Aromatenbildung unterdrücken und die niederer Olefine erhöhen. Es ist offenkundig, dass eine solche Verfahrensweise mit technischen Problemen hinsichtlich Raum-Zeit-Ausbeute, Gasrückführung und Trennung der Reaktionsprodukte von den gasförmigen Inertstoffen belastet ist.

Überraschenderweise wurde nun gefunden, dass die katalytische Umwandlung von Methanol und/oder Dimethylether in Gegenwart des Zeolithen PSH-3 ein Kohlenwasserstoffgemisch liefert, dass einen niedrigen Aromatengehalt aufweist.

Die erfindungsgemässe Anwendung des Zeoliths PSH-3 als Katalysator zur Umwandlung von Methanol und/oder Dimethylether kann entweder diskontinuierlich, halbkontinuierlich oder kontinuierlich durchgeführt werden. Dabei ist es unerheblich, ob der Katalysator fest oder beweglich angeordnet ist.

Die nachfolgende Erläuterung bezieht sich auf Methanol als Einsatzmaterial. Es ist jedoch zu berücksichtigen, dass mit gleichem Erfolg auch Dimethylether oder Dimethylether/Wasser-Mischungen eingesetzt werden können.

Der Methanoleinsatz kann aus reinem Methanol oder aus Wasser- und oxygenathaltigem technischem Rohmethanol bestehen. Ebenso kann eine zusätzliche Verdünnung des Einsatzes mit Wasser vorgenommen werden.

Das Verfahren ist durch folgende Reaktionsbedingungen gekennzeichnet

| Bereich | | bevorzugt | besonders bevorzugt |
|---|---|---|---|
| Temperatur (°C) | 200-600 | 250-450 | 300-400 |
| Druck (bar) | 1-100 | 1-50 | 1-10 |
| Katalysatorbelastung ($g\ CH_3OH/l \cdot h$) | 10-10000 | 100-5000 | 500-2000 |

Es kann notwendig sein, den verwendeten Katalysator auf Basis Zeolith PSH-3 nach einer gewissen Betiebsdauer von desaktivierenden Ablagerungen zu befreien. Diese Regenerierung wird zweckmässigerweise durch Zufuhr einer sauerstoffhaltigen Atmosphäre (z.B. Luft oder Luft/Stickstoff-Gemische) zum Katalysator bei erhöhter Temperatur nach bekannten Techniken durchgeführt. Die Regeneriertemperatur sollte 600° C nicht übersteigen.

Ebenso kann anstelle von Methanol und/oder Dimethylether direkt Synthesegas als umzuwandelndes Einsatzmaterial verwendet werden, sofern der Zeolith PSH-3 mit für die Methanol- bzw. Fischer-Tropsch-Synthese katalytisch geeigneten Metallen belegt ist.

Das erfindungsgemässe Aluminosilicat kann so in Verbindung mit Katalysatoren eingesetzt werden, die für die Kohlenoxidhydrierung geeignet sind. Während die bei der üblichen Ausführung der Kohlenoxidhydrierung (Fischer-Tropsch-Synthese mit metallhaltigen Katalysatoren) entstehende Fraktion der längerkettigen Kohlenwasserstoffe ($C_{5+}$) oft bis über 20 bis 25 C-Atome im Molekül aufzeigt und zum grossen Teil geradkettig aufgebaut ist, liefert die Anwendung des erfindungsgemässen Aluminosilicats als Cokatalysator bei der katalytischen Kohlenoxidhydrierung neben anderen Produktbestandteilen ein $C_{5+}$-Kohlenwasserstoffgemisch, das überwiegend aus Molekülen mit 5 bis 12 C-Atomen besteht. Dieser kettenlängenbegrenzende Effekt des erfindungsgemässen Aluminosilicats ist bei entsprechender Führung der Reaktion mit einer Bildung verzweigter Kohlenwasserstoffe und gegebenenfalls weiteren, zu Aromaten führenden Reaktionen verbunden, wodurch z.B. ein so hergestelltes Kohlenwasserstoffgemisch zur Weiterverarbeitung in Ottokraftstoff wesentlich besser geeignet ist als das bei der üblichen Ausführung der Fischer-Tropsch-Synthese entstehende Kohlenwasserstoffgemisch.

Anhand der folgenden Beispiele soll die Erfindung noch näher erläutert werden. %-Angaben beziehen sich – soweit nicht anders vermerkt ist – auf Gew.-%.

*Beispiel 1:*

In einem 2-l-Stahl-Autoklav mit Rühreinsatz wird eine Mischung aus 217 ml Wasserglas (27% $SiO_2$ und 8% $Na_2O$) und 316 ml Wasser mit 49,5 g Hexamethylenimin versetzt und intensiv gerührt. In diese Mischung lässt man langsam unter Rühren eine Lösung aus 9,22 g Al-Sulfat, 23,4 g $H_2SO_4$ (konz.) und 460 ml Wasser einfliessen.

Nachdem das entstandene Reaktionsgel homogenisiert worden ist, lässt man bei 150° C innerhalb von 70 h kristallisieren.

Nach Ende der Reaktionszeit wird der Feststoff von der flüssigen Phase abgetrennt, ausgewaschen, bis der pH-Wert bei 7 bis 9 lag, und bei 120° C getrocknet.

Der Zeolith hatte die in der Tabelle angegebenen Röntgenbeugungsdaten und nach dem Calcinieren bei 500° C folgend Zusammensetzung

$$0{,}4\ Na_2O \cdot Al_2O_3 \cdot 55\ SiO_2.$$

| d (Å) | 100 I/$I_0$ |
|---|---|
| 12,63 | 100 |
| 10,92 | 30 |
| 8,84 | 60 |
| 6,86 | 5 |
| 6,15 | 40 |
| 5,50 | 15 |
| 4,39 | 15 |
| 4,09 | 20 |
| 3,91 | 30 |
| 3,75 | 5 |
| 3,56 | 3 |
| 3,41 | 100 |
| 3,30 | 2 |
| 3,19 | 3 |
| 3,11 | 2 |
| 2,836 | 2 |
| 2,694 | 3 |
| 2,592 | 2 |
| 2,392 | 2 |
| 2,206 | 1 |
| 2,122 | 1 |
| 2,036 | 2 |
| 1,973 | 5 |
| 1,873 | 3 |
| 1,855 | 5 |

*Beispiel 2:*

100 g Zeolith PSH-3 in der calcinierten Form werden mit 100 ml 0,5N HCl bei 80 °C 5 h gerührt. Der Feststoff wird abgetrennt und gewaschen. Der Austausch wird noch zweimal wiederholt. Die H-Form des Zeoliths kann als Katalysator für die Herstellung von Kohlenwasserstoffen aus Methanol eingesetzt werden.

*Beispiel 3:*

Der Ansatz wie in Beispiel 1 wurde bei 130° C innerhalb von 144 h kristallisiert. Das erhaltene kristalline Produkt wies die identischen Röntgendaten wie der Zeolith PSH-3 aus Beispiel 1 auf.

*Beispiel 4:*

In einem 5-l-Stahl-Autoklav mit Rührereinsatz wird eine Mischung aus 650 g Kieselsol, 790 ml Wasser und 78 g NaOH mit 124 g Hexamethylenimin versetzt. In diese Mischung wird langsam eine Lösung aus 23 g Al-Sulfat, 58,5 g $H_2SO_4$ (konz.) und 1150 ml $H_2O$ gegeben.

Das Reaktionsgemisch lässt man bei 150° C 5 d unter Rühren kristallisieren.

Der kristalline Feststoff wird abgetrennt, ausgewaschen, bis der pH-Wert im ablaufenden Wasser 8 bis 9 betrug. Der Zeolith hatte nach dem Calcinieren die Röntgenbeugungsdaten der Tabelle 1.

*Beispiel 5:*

Ansatz wie in Beispiel 1, nur wurde hier die Menge des Hexamethylenimins um 50% reduziert. Die Kristallisation erfolgte bei 140° C innerhalb von 96 h.

Das kristalline Endprodukt wies hauptsächlich die Röntgendaten der Tabelle 1 auf, neben geringen Mengen an Quarz.

*Beispiel 6:*

Ansatz wie in Beispiel 1, nur wurde hier die Menge des Al-Sulfats auf 15 g erhöht. Das kristalline Endprodukt wies nach der Calcinierung folgende oxidische Zusammensetzung auf

$$0{,}2\ Na_2O \cdot Al_2O_3 \cdot 41\ SiO_2$$

Die Röntgendaten stimmten im wesentlichen mit denen der Tabelle 1 überein.

Die nachfolgenden Beispiele 7 bis 8 betreffen die Verwendung des nach den Beispielen 1 bis 6 hergestellten Zeoliths PSH-3 als Katalysator für die Umwandlung von Methanol und/oder Dimethylether in Kohlenwasserstoffe.

*Beispiel 7:*

Ein nach Beispiel 1 hergestellter und nach Beispiel 2 dekationisierter Zeolith PSH-3 wurde mit 15% Bentonit versetzt und mit Wasser zu einer extrudierbaren Masse verarbeitet. Nach Extrusion, Trocknung bei 120° C und Calcinierung bei 500° C wurden Katalysatorformkörper erhalten. Die Ausprüfung dieses Katalysatormaterials erfolgte ohne Anwendung von Druck in einem elektrisch beheiztem Rohrreaktor (d = 20 mm, Katalysatorfüllhöhe 160 mm), der mit einer Vorheizzone sowie Einlässen für Stickstoff und Methanol versehen war.

Der Katalysator wurde unter Stickstoff auf eine Reaktionstemperatur von 370° C gebracht und dann mit gasförmigem Methanol in einer Menge von 790 g pro Liter Katalysator und pro Stunde beaufschlagt.

Der Methanolumsatz betrug bei diesem Versuch 94,5 Gew.-%. Neben Wasser und nichtumgesetztem Methanol wurde ein organisches Reaktionsprodukt der folgenden Zusammensetzung erhalten (Angaben in Gew.-%)

| | |
|---|---|
| Methan | 0,9 |
| Ethan | 0,1 |
| Ethen | 2,7 |
| Propan | 2,1 |
| Propen | 11,0 |
| n-Butan | 0,7 |
| i-Butan | 4,2 |
| n-Buten | 7,5 |
| i-Buten | 3,4 |
| $C_5^+$-Aliphaten (inkl. Olefine) | 40,5 |
| $C_6^+$-Aromaten | 13,0 |
| Dimethylether | 13,9 |
| Summe | 100,0 |

Da der bei der Reaktion entstehende Dimethylether rezirkulierbar ist, errechnet sich für die Summe der $C_5^+$-Kohlenwasserstoffe eine Kohlenstoff-Selektivität von ca. 46%. Unter Einbeziehung der $C_4^-$-Kohlenwasserstoffe erhöht sich dieser Wert auf ca. 84%. Hierdurch wird offenbar, dass sich Zeolith PSH-3 in besonderer Weise für die Herstellung eines olefinreichen, aliphatischen Kohlenwasserstoffgemisches aus Methanol eignet.

*Beispiel 8:*

Dieses Beispiel bezieht sich auf die Umwandlung einer Methanol/Dimethylether/Wasser-Gleichgewichtsmischung in überwiegend aliphatisch-olefinische Kohlenwasserstoffe.

Ein Strom von 50 ml Methanol pro Stunde wurde in verdampfter Form ohne Anwendung von Druck bei einer Temperatur von 280° C über eine Katalysatorschüttung bestehend aus 100 ml gefälltem und granuliertem Aluminiumoxid geleitet. Aus der Literatur ist bekannt, dass Aluminiumoxid die Umsetzung von Methanol zu Dimethylether katalysiert (s. beispielsweise J.B. Senderens, „Ann. Chim. Phys.", 25, 509 [1912]). Das den Reaktor verlassende Produktgemisch wurde ohne weitere Trennung über einen nach Beispielen 1 und 2 hergestellten und mit einem Zusatz von 20% handelsüblichen Bentonit nach bekannten Techniken granulierten Katalysator auf Basis Zeolith PSH-3 geleitet.

Die Kontaktbelastung betrug 800 g Einsatz pro Liter Katalysator und pro Stunde; die Umwandlungstemperatur betrug 370° C.

Der den Reaktor verlassende Produktstrom setzte sich wie folgt zusammen (ohne Berücksichtigung des Reaktionswassers)

| | |
|---|---|
| Methan | 1,2 |
| Ethan | 0,1 |
| Ethen | 3,4 |
| Propan | 2,0 |
| Propen | 15,2 |
| Butan | 5,0 |
| Butene | 7,9 |
| $C_5^+$-Kohlenwasserstoffe (P+O) | 39,4 |
| Benzol | 0,4 |
| Toluol | 0,4 |
| $C_8$-Aromaten | 1,2 |
| $C_9^+$-Aromaten | 6,9 |
| Dimethylether | 16,9 |
| Summe | 100,0 |

Im $C_5^+$-Anteil können auch kleine Anteile sauerstoffhaltiger Verbindungen enthalten sein.

Da der entstandene Dimethylether zurückgeführt werden kann, errechnet sich für die aliphatischen Kohlenwasserstoffe im Benzinsiedebereich ($C_5^+$, P + O) eine Kohlenstoffselektivität von ca. 47%. Dieser Wert erhöht sich auf ca. 83%, sofern auch $C_3$- und $C_4$-Kohlenwasserstoffe hinzugenommen werden. Aus den letzteren lassen sich nach bekannten Techniken (Alkylierungsprozesse) ebenfalls Vergaserkraftstoffe gewinnen.

*Beispiel 9:*

Zur Herstellung eines Katalysators zur Kohlenoxidhydrierung wurde eine Suspension aus 18,4 g des erfindungsgemässen Aluminosilicats in 250 ml einer wässerigen Lösung mit 18,4 g Kaliwasserglas und 70,1 g $Fe(NO_3)_3 \times 9H_2O$ in der Siedehitze mit 160 ml 11%iger Ammoniaklösung versetzt. Der Niederschlag wurde abfiltriert, mit Wasser neutral gewaschen und 4 h bei 150° C im Vakuum (20 Torr) getrocknet. Nach Mahlung wurde die Masse mit Graphit und Bentonit zu zylindrischen 5 × 5-mm-Tabletten gepresst. Unter der Annahme vollständiger Ausfällung des Eisens und quantitativer Sorption des Kaliwasserglases durch den Niederschlag berechnet man folgende Katalysatorzusammensetzung

| | |
|---|---|
| Fe | 31,5 Gew.-% |
| Erfindungsgemässes Aluminosilicat | 29,6 Gew.-% |
| Kaliwasserglas | 8,9 Gew.-% |
| Bentonit | 20,0 Gew.-% |
| Graphit | 10,0 Gew.-% |
| Summe | 100,0 Gew.-% |

50 ml dieses das erfindungsgemässe Aluminosilicat enthaltenden Kohlenoxidhydrierungskatalysators wurden zur besseren Wärmeabfuhr mit 450 ml eines überwiegend aus Siliciumcarbid bestehenden Materials vermischt und in einen mit Wärmeträgeröl thermostatisierten Rohrreaktor von 25 mm Durchmesser und 150 cm Länge gefüllt. Die Katalysatorfüllung wurde zunächst 14 h lang bei 300° C, Normaldruck und einer Gasbelastung von 1000 Nl/(h × 1 Katalysator) sowie anschliessend mit Kohlenmonoxide 8 h lang bei 300° C, Normaldruck und einer Gasbelastung von 1000 Nl/(h × 1 Katalysator) behandelt. Die hier und in der folgenden, die Ergebnisse der Kohlenoxidhydrierung enthaltenden Tabelle angegebenen Gasbelastungen beziehen sich stets auf die eingesetzten 50 ml des oben beschriebenen Katalysators. Die kettenlängenbegrenzende Wirkung des erfindungsgemässen Aluminosilicats geht aus den Angaben der beiden Beispiele hervor, wonach 95 Gew.-% der $C_{5+}$-Fraktion $\leqslant 12$ C-Atome im Molekül aufweisen.

*Tabelle*

| Beispiel | 9a | 9b |
|---|---|---|
| Versuchsdauer (h) | 12 | 12 |
| Rohrwandtemperatur (°C) | 300 | 310 |
| Max. Temp. im Katalysatorbrett (°C) | 307 | 324 |
| Druck ($H_2$+CO) (bar) | 26,0 | 25,9 |
| $H_2$: CO-Einsatzverhältnis (–) | 1,05: 1 | 1,19: 1 |
| Gasbelastung ($H_2$+CO)Nl/(h×1 Katalysator) | 1924 | 3029 |
| Umsatz $H_2$ (%) | 62,7 | 58,3 |
| Umsatz CO | 62,4 | 58,5 |
| Umsatz $H_2$+CO | 62,4 | 58,5 |
| Selektivität, bezogen auf umgesetztes CO (%) | | |
| $CH_4$ | 9,7 | 14,4 |
| $C_{1+}$ | 71,5 | 69,3 |
| $CO_2$ | 28,5 | 30,7 |
| Selektivität, bezogen auf $C_{1+}$ | | |
| $CH_4$ | 13,6 | 20,7 |
| $C_2H_4$ | 0,9 | 1,1 |
| $C_2H_6$ | 7,1 | 9,7 |
| $C_3H_6$ | 6,9 | 7,5 |
| $C_3H_8$ | 4,7 | 5,7 |
| $C_4H_8$ | 5,8 | 6,5 |
| $C_4H_{10}$ | 2,6 | 3,2 |
| $C_{5+}$ | 58,4 | 45,5 |

| | | |
|---|---|---|
| Verteilung der Kettenlänge in $C_{5+}$: | | |
| Anteil bis n-$C_8$-Paraffin (Gew.-%) | 67 | 67 |
| Anteil bis n-$C_{12}$-Paraffin | 95 | 95 |
| Anteil bis n-$C_{20}$-Paraffin | 100 | 100 |
| Spezif. Ausbeute $C_{1+}$g/$Nm^3$ umgesetztes Synthesegas | 133 | 119 |
| Raum-Zeit-Ausbeute $C_{1+}$ g/(h×1 Katalysator) | 256 | 361 |

## Patentansprüche

1. Kristallines Aluminosilicat mit einer Zusammensetzung entsprechend der Formel (in Molverhältnissen der Oxide):

$$M_{2/n}O \cdot Al_2O_3 \cdot (20\text{-}150)\ SiO_2$$

wobei M ein Kation der Wertigkeit n ist und dem folgenden Pulverröntgendiagramm:

| d-Wert (Å) | Intensität |
|---|---|
| 12,63 | sehr stark |
| 10,92 | mittel |
| 8,84 | sehr stark |
| 6,86 | schwach |
| 6,15 | stark |
| 5,50 | schwach |
| 4,91 | schwach |
| 4,60 | schwach |
| 4,39 | schwach |
| 4,09 | schwach |
| 3,91 | mittel |
| 3,75 | schwach |
| 3,56 | schwach |
| 3,41 | sehr stark |
| 3,30 | schwach |
| 3,19 | schwach |
| 3,11 | schwach |
| 2,836 | schwach |
| 2,694 | schwach |
| 2,592 | schwach |
| 2,392 | schwach |
| 2,206 | schwach |
| 2,122 | schwach |
| 2.036 | schwach |
| 1,973 | schwach |
| 1,873 | schwach |
| 1,855 | schwach |

2. Verfahren zur Herstellung von kristallinen Aluminosilicaten nach Anspruch 1, dadurch gekennzeichnet, dass man ein Reaktionsgemisch aus geeigneten oxidischen Verbindungen und Hexamethylenimin einer Zusammensetzung mit den oxidischen Molverhältnissen

$SiO_2/Al_2O_3$ = 10-200
$OH^-/SiO_2$ = 0,05-1,0
$R/Na_2O$ = 0,5-5,0
(R = Hexamethylenimin $(CH_2)_6NH$)

bei Temperaturen zwischen 80 und 180°C über einen Zeitraum von 12 bis 144 h kristallisieren lässt, das kristalline Produkt von der Mutterlauge abtrennt, wäscht und trocknet.

3. Verwendung von kristallinem Aluminosilicat gemäss Anspruch 1 als Katalysator für die katalytische Umwandlung von Methanol und/oder Dimethylether in Kohlenwasserstoffe.

4. Verwendung gemäss Anspruch 3, dadurch gekennzeichnet, dass das kristalline Aluminosilicat in ionenausgetauschter Form zum Einsatz gelangt.

5. Verwendung gemäss Anspruch 3, dadurch gekennzeichnet, dass das kristalline Aluminosilicat in der H-Form eingesetzt wird.

6. Verwendung gemäss Anspruch 3, dadurch gekennzeichnet, dass das kristalline Aluminosilicat in einer metallausgetauschten Form zum Einsatz gelangt.

7. Verwendung von kristallinem Aluminosilicat gemäss Anspruch 1 in Verbindung mit anderen Katalysatoren für die Kohlenoxidhydrierung.

8. Verwendung von kristallinem Aluminosilicat gemäss Anspruch 1 in ionenausgetauschter Form und in Verbindung mit Katalysatoren für die Kohlenoxidhydrierung.

## Claims

1. A crystalline aluminosilicate having a composition corresponding to the formula (in molar ratios of the oxides)

$$M_{2/n}O \cdot Al_2O_3 \cdot (20\text{-}150)\ SiO_2$$

wherein

M is a cation of the valency n and having the following powder X-ray diagram

| d-value (Å) | intensity |
|---|---|
| 12.63 | very strong |
| 10.92 | medium |
| 8.84 | very strong |
| 6.86 | weak |
| 6.15 | strong |
| 5.50 | weak |
| 4.91 | weak |
| 4.60 | weak |
| 4.39 | weak |
| 4.09 | weak |
| 3.91 | medium |
| 3.75 | weak |
| 3.56 | weak |
| 3.41 | very strong |
| 3.30 | weak |
| 3.19 | weak |
| 3.11 | weak |
| 2.836 | weak |
| 2.694 | weak |
| 2.592 | weak |
| 2.392 | weak |
| 2.206 | weak |
| 2.122 | weak |
| 2.036 | weak |
| 1.973 | weak |
| 1.873 | weak |
| 1.855 | weak |

2. Process for the production of crystalline aluminosilicates according to Claim 1, characterised in that a reaction mixture of suitable oxidic compounds and hexamethylene imine of a composition having the oxidic molar ratios

$SiO_2/Al_2O_3$ = 10-200
$OH^-/SiO_2$ = 0.05-1.0
$R/Na_2O$ = 0.5-5.0
(R = hexamethylene imine $(CH_2)_6MH$)

is allowed to crystallise at temperatures of between 80 and 180°C over a period of 12 to 144 h, and the crystalline product is separated from the mother liquor, washed and dried.

3. Use of crystalline aluminosilicate according to Claim 1 as a catalyst for the catalytic conversion of methanol and/or dimethyl ether into hydrocarbons.

4. Use according to Claim 3, characterised in that the crystalline aluminosilicate is used in an ion-exchanged form.

5. Use according to Claim 3, characterised in that the crystalline aluminosilicate is used in the H-form.

6. Use according to Claim 3, characterised in that the crystalline aluminosilicate is used in a metal-exchanged form.

7. Use of crystalline aluminosilicate according to Claim 1 in conjunction with other catalysts for the hydrogenation of carbon monoxide.

8. Use of crystalline aluminosilicate according to Claim 1 in an ion-exchanged form and in conjunction with catalysts for the hydrogenation of carbon monoxide.

**Revendications**

1. Silicate d'aluminium cristallin ayant une composition correspondant à la formule suivante (en rapports molaires des oxydes)

$$M_{2/n}O \cdot Al_2O_3 \cdot (20\text{-}150)\ SiO_2$$

où M représente un cation de la valence n, avec le diagramme de diffraction des rayons X de poudres ci-après

| Valeur d (Å) | Intensité |
|---|---|
| 12,63 | très forte |
| 10,92 | moyenne |
| 8,84 | très forte |
| 6,86 | faible |
| 6,15 | forte |
| 5,50 | faible |
| 4,91 | faible |
| 4,60 | faible |
| 4,39 | faible |
| 4,09 | faible |
| 3,91 | moyenne |
| 3,75 | faible |
| 3,56 | faible |
| 3,41 | très forte |
| 3,30 | faible |
| 3,19 | faible |
| 3,11 | faible |
| 2,836 | faible |
| 2,694 | faible |
| 2,592 | faible |
| 2,392 | faible |
| 2,206 | faible |
| 2,122 | faible |
| 2,036 | faible |
| 1,973 | faible |
| 1,873 | faible |
| 1,855 | faible |

2. Procédé de préparation de silicates d'aluminium cristallins suivant la revendication 1, caractérisé en ce qu'on laisse cristalliser un mélange réactionnel constitué de composés d'oxydes appropriés et d'hexaméthylène-imine, ce mélange ayant la composition suivante (avec les rapports molaires d'oxydes)

$SiO_2/Al_2O_3$ = 10-200
$OH^-/SiO_2$ = 0,05-1,0
$R/Na_2O$ = 0,5-5,0
(R = hexaméthylène-imine $(CH_2)_6NH$)

à des températures comprises entre 80 et 180°C pendant un laps de temps de 12 à 144 h, en ce qu'on sépare le produit cristallin de la liqueur mère, qu'on le lave et qu'on le sèche.

3. Utilisation du silicate d'aluminium cristallin suivant la revendication 1 comme catalyseur pour la transformation catalytique du méthanol et/ou de l'éther diméthylique en hydrocarbure.

4. Utilisation suivant la revendication 3, caractérisée en ce que le silicate d'aluminium cristallin est utilisé sous une forme échangée par des ions.

5. Utilisation suivant la revendication 3, caractérisée en ce que le silicate d'aluminium cristallin est utilisé sous la forme H.

6. Utilisation suivant la revendication 3, caractérisée en ce que le silicate d'aluminium cristallin est utilisé sous une forme échangée par un métal.

7. Utilisation du silicate d'aluminium cristallin suivant la revendication 1 en combinaison avec d'autres catalyseurs pour l'hydrogénation de l'oxyde de carbone.

8. Utilisation du silicate d'aluminium cristallin suivant la revendication 1 sous une forme échangée par des ions et en combinaison avec des catalyseurs pour l'hydrogénation de l'oxyde de carbone.